(19) Europäisches Patentamt
European Patent Office
Office européen des brevets

(11) **EP 0 649 300 B1**

(12) **FASCICULE DE BREVET EUROPEEN**

(45) Date de publication et mention
de la délivrance du brevet:
**05.03.1997 Bulletin 1997/10**

(51) Int. Cl.$^6$: **A61K 6/00**, A61L 31/00,
A61L 27/00

(21) Numéro de dépôt: 93913091.0

(22) Date de dépôt: 04.06.1993

(86) Numéro de dépôt international:
**PCT/FR93/00535**

(87) Numéro de publication internationale:
**WO 93/24097 (09.12.1993 Gazette 1993/29)**

(54) **UTILISATION DE MELANGES DE POLYMERES DERIVES DES ACIDES LACTIQUES DANS LA PREPARATION DE MEMBRANES BIORESORBABLES POUR LA REGENERATION TISSULAIRE GUIDEE, NOTAMMENT EN PARODONTOLOGIE**

VERWENDUNG VON MILCHSÄUREPOLYMEREN ZUR HERSTELLUNG VON BIORESORBIERBAREN MEMBRANEN FÜR DIE GESTEUERTE GEWEBSGENERATION INSBESONDERE FÜR DIE PARODONTOLOGIE

USE OF MIXTURES OF POLYMERS DERIVED FROM LACTIC ACIDS IN THE PREPARATION OF BIORESORBABLE MEMBRANES FOR GUIDED TISSUE REGENERATION, PARTICULARLY IN PERIODONTOLOGY

(84) Etats contractants désignés:
**AT BE CH DE DK ES FR GB GR IE IT LI LU MC NL PT SE**

(30) Priorité: **04.06.1992 FR 9206797**

(43) Date de publication de la demande:
**26.04.1995 Bulletin 1995/17**

(73) Titulaire: **CENTRE NATIONAL DE LA RECHERCHE SCIENTIFIQUE (CNRS) 75016 Paris (FR)**

(72) Inventeurs:
• **VERT, Michel**
  **F-76130 Mont-Saint-Aignan (FR)**

• **MAUDUIT, Jacques**
  **F-76730 Bacqueville-en-Caux (FR)**
• **FRANK, Robert**
  **F-67000 Strasbourg (FR)**
• **ROBERT, Pierre**
  **F-67400 Illkirch-Gruffenstaden (FR)**

(74) Mandataire: **Tonnellier, Jean-Claude et al Nony & Associés, 29, rue Cambacérès 75008 Paris (FR)**

(56) Documents cités:
**EP-A- 0 475 077          WO-A-92/15340
FR-A- 2 612 392          FR-A- 2 635 685**

## Description

La présente invention a pour objet l'obtention de nouvelles membranes biorésorbables à base de poly(acides lactiques) destinées à être implantées pour favoriser la régénération du parodonte et/ou des maxillaires selon la technique de régénération tissulaire guidée, notamment en parodontologie.

Les maladies parodontales sont très fréquentes chez l'homme et constituent, avec la carie dentaire, la cause principale de la perte des dents.

On sait que les dents comportent une partie visible (la couronne) et une partie radiculaire insérée dans l'os maxillaire. Le principal constituant de la dent, appelé dentine, est recouvert d'émail au niveau de la couronne, et de cément au niveau de la racine. La ligne de jonction entre le cément et l'émail, appelée région cervicale, est normalement recouverte par la muqueuse gingivale. Les dents sont fixées au tissu osseux des maxillaires par l'intermédiaire d'un tissu conjonctif fibreux, appelé ligament alvéolo-dentaire. On englobe sous la notion de parodonte l'ensemble des tissus de soutien de la dent, c'est-à-dire la muqueuse gingivale, le ligament alvéolo-dentaire, le cément et l'os alvéolaire.

Les parodontites, qui sont provoquées par des bactéries principalement anaérobies, conduisent à une destruction des tissus de soutien dentaire, notamment la gencive, l'os alvéolaire, le ligament alvéolo-dentaire et le cément. Ces destructions tissulaires débutent par une prolifération bactérienne à prédominance anaérobique et Gram négatif, créant ainsi une poche parodontale qui va s'approfondir progressivement en migrant, en direction de l'extrémité radiculaire, entre la gencive et la surface radiculaire.

Le traitement chirurgical actuel des maladies parodontales est basé sur le principe de la régénération tissulaire guidée. Il consiste, après avoir réalisé un lambeau gingival muco-périosté, à cureter la surface radiculaire et à recouvrir cette surface par une barrière membranaire, de telle sorte que la surface curetée soit isolée des tissus épithéliaux et conjonctifs de la gencive et puisse être colonisée par du tissu conjonctif provenant du ligament alvéolo-dentaire. Au bout de quelques semaines, on peut alors obtenir une régénération du parodonte, sans récidive de poche parodontale. Les premiers essais de régénération tissulaire guidée ont été effectués à l'aide de membranes de polytétrafluoro-éthylène (voir par exemple S. Nyman et al., J.Clin.Periodontol. 9:290-296, 1982). Un inconvénient majeur est que ce matériau n'est pas résorbable et nécessite donc une deuxième intervention chirurgicale, longue et désagréable pour le patient et risquant de favoriser une nouvelle inflammation, pour retirer la membrane implantée.

On a proposé l'utilisation de divers matériaux biodégradables permettant d'éviter la seconde intervention chirurgicale destinée à retirer la membrane implantée, par exemple l'utilisation de collagène; voir par exemple N. M. Blumenthal, J. Periodontol. 59 : 830-836, 1988. Cependant, l'utilisation de collagène fait courir des risques de réactions immunologiques. On a également proposé l'utilisation d'un copolymère acide glycolique-acide lactique commercialisé sous la marque Vicryl (voir U. Zappa, Rev. Mens. Suisse Odontostomatol., Vol. 101 : 10 1991, pages 13.25-13.26). Toutefois, il résulte de l'expérience de la demanderesse que les implants à base d'acide polyglycolique peuvent être à l'origine de réactions inflammatoires indésirables.

Le document WO-A-9215340, publié après la date de priorité revendiquée dans la présente demande, décrit un matériau biorésorbable pouvant être utilisé dans la régénération du tissu parodontal. Ce matériau est constitué par le mélange d'un polymère biorésorbable et d'un plastifiant, ce dernier étant choisi parmi divers esters tels que les oligomères d'acide lactique à terminaison ester d'éthyle ou les esters de l'acide lactique avec le glycérol.

Le document EP-A-0475077 décrit une membrane en matériau biodégradable pour la régénération osseuse. Cette membrane est constituée d'un matériau polymère dont la température de transition vitreuse est dans la gamme de -30 à +200°C et dont la masse moléculaire est dans la gamme de 5000 à 1000000.

Le document FR-A-2635685 décrit, comme matériau pour la régénération du parodonte, un copolymère lactide/caprolactone ou lactide/glycolide ayant une masse moléculaire de 40000 à 500000.

On a maintenant découvert qu'il est possible d'obtenir des membranes utilisables pour la régénération tissulaire guidée, qui sont biorésorbables et qui ne provoquent pas de réactions inflammatoires ou allergiques.

La présente invention a pour objet l'utilisation, dans la préparation d'une membrane implantable sous la forme d'un film mince à base de poly(acide lactique), destinée à favoriser la régénération du parodonte ou de l'os maxillaire selon la technique de régénération tissulaire guidée, d'un mélange d'un premier poly(acide lactique) amorphe ayant une masse moléculaire moyenne élevée au moins égale à 20.000, et en particulier au moins égale à 50.000 environ et d'un second poly(acide lactique) amorphe ayant une masse moléculaire moyenne faible non supérieure à 5.000 environ, ledit mélange comportant une proportion suffisante dudit second poly(acide lactique) pour que la membrane se présente sous la forme d'un film ayant le degré de souplesse désiré à une température non supérieure à 37°C.

Pour obtenir des polymères intrinsèquement amorphes, il suffit de préparer les polymères à partir d'un produit de départ contenant un mélange d'acides D- et L-lactiques (sous forme d'acide ou sous forme de lactide). La proportion de motifs D-lactiques au moins suffisante pour que le polymère soit amorphe peut être facilement déterminée dans chaque cas par de simples expériences de routine. Le plus souvent, on utilisera des polymères contenant de 30 à 70 % de motifs dérivés de l'acide D-lactique.

Les poly(acides lactiques), ou PLA, de faibles masses moléculaires jouent le rôle de plastifiant dans les mélanges utilisés selon l'invention, et la souplesse du film augmente avec la teneur en PLA de faibles masses moléculaires. Les mélanges de PLA ont une température de transition vitreuse intermédiaire entre celle du PLA de faible masse moléculaire et celle, plus élevée, du PLA ayant une masse moléculaire supérieure à 20.000. Plus la proportion de PLA de faible masse moléculaire augmente et plus la température de transition vitreuse du mélange diminue, cet effet étant d'autant plus marqué que le PLA de masse moléculaire inférieure à 5.000 a une masse moléculaire plus faible. On peut donc régler à volonté le degré de souplesse des membranes, à une température donnée, en faisant varier la proportion du PLA de faible masse moléculaire, par exemple de 10 à 60 % en poids.

On peut ainsi déterminer facilement, par de simples expériences de routine, les mélanges qui conviennent pour que la membrane ait un degré de souplesse suffisant pour faciliter l'implantation et le maintien à l'aide de points de suture, tout en évitant le traumatisme des tissus mous. Le plus souvent, les mélanges convenables contiennent de 20 à 60 % en poids du PLA de faible masse moléculaire, en particulier de 20 à 50 %, et notamment de 25 à 50 %, dans le cas de la régénération du parodonte. Dans le cas de régénération osseuse du maxillaire destinée à accompagner la mise en place d'un implant ostéointégré, après extraction de la dent accompagnée d'une perte osseuse, il est souhaitable que la membrane ne soit pas trop souple et qu'elle se dégrade moins rapidement. On utilisera alors de préférence des membranes ayant des teneurs peu élevées, par exemple de 10 à 20 %, en PLA de faible masse moléculaire.

Les masses moléculaires moyennes des polymères sont déterminées par exemple en solution, par chromatographie par perméation de gel (GPC), par comparaison avec des polymères étalons, tels que des étalons de polystyrène.

Les masses moléculaires (MM) peuvent être déterminées en nombre ou en poids. Par exemple, la limite inférieure de 20.000 pour le polymère de MM élevée est de préférence une MM en nombre, et la limite supérieure de 5.000 pour le polymère de faible MM est de préférence une MM en poids.

De préférence, les polymères utilisés ont un indice de polymolécularité de dépassant pas 3.

Le matériau des membranes utilisées selon l'invention est donc caractérisé en GPC par une répartition bimodale des masses moléculaires.

Pour préparer les PLA de masses moléculaires relativement élevées, on peut opérer de façon connue par ouverture de cycle de lactides. On opère sur un mélange de D- et L-lactides dans les proportions choisies.

Les PLA de faibles masses moléculaires peuvent également être obtenus selon des méthodes connues, notamment par polycondensation de mélanges d'acides L- et D-lactiques, par exemple par polycondensation de l'acide D,L-lactique.

En outre, des PLA de diverses masses moléculaires peuvent être trouvés dans le commerce.

Pour préparer les membranes utilisées selon l'invention, on peut solubiliser les deux PLA dans un solvant, par exemple l'acétone, le dioxane, le chloroforme ou le dichlorométhane, puis évaporer le solvant, éventuellement sous pression réduite, en évitant une évaporation trop rapide qui favoriserait la formation de bulles et donc d'irrégularités en surface. Les quantités de polymères peuvent être ajustées pour obtenir un film d'épaisseur appropriée. Cette épaisseur peut en outre être modifiée par calandrage à une température supérieure à la température de transition vitreuse du mélange de polymères.

Les membranes utilisées selon l'invention ont par exemple une épaisseur pouvant aller de 0,05 à 0,5 mm, et en particulier de 0,1 à 0,3 mm. Elles peuvent être stérilisées par exemple par irradiation.

Il est bien entendu possible d'incorporer dans le film divers additifs utiles qui seront libérés progressivement lors de la résorption de la matrice polymère. Parmi les additifs pouvant être dispersés dans la matrice polymère de la membrane, on peut citer par exemple diverses substances actives telles que des antibiotiques, des anti-inflammatoires, des agents favorisant la néoformation osseuse, etc.

Ces substances actives peuvent être incorporées dans la solution servant à la préparation de la membrane. Elles peuvent aussi être incorporées sous forme de poudres fines par mélange et malaxage avec le matériau de la membrane à température supérieure à la température de ramollissement du mélange de polymères. Le mélange est ensuite mis sous la forme d'un film par calandrage.

La membrane biorésorbable utilisée selon l'invention est mise en place selon les techniques connues de régénération tissulaire guidée destinée au traitement des maladies parodontales, après élimination du tissu inflammatoire parodontal. Au bout de 6 à 12 semaines en général, on peut observer une amélioration importante, incluant une régénération de la gencive, du ligament, du cément, et de l'os alvéolaire. La membrane biorésorbable utilisée selon l'invention est également applicable à une régénération osseuse guidée, selon les techniques connues, dans les cas d'implants dentaires ostéointégrés intramaxillaires.

Les membranes utilisées selon l'invention sont progressivement résorbées, la résorption étant d'autant plus rapide que notamment la proportion de PLA de faible masse moléculaire est plus importante et que cette masse moléculaire est plus faible. Pour un matériau de composition et d'épaisseur données, la vitesse de dégradation et de résorption peut être estimée par exemple à l'aide d'un modèle de dégradation in vitro, par exemple dans une solution tampon, à température ambiante, ou à 37°C. Il est donc facile de régler la composition du mélange de PLA en fonction de la vitesse de résorption souhaitée, notamment de façon que la membrane ne soit pas totalement résorbée avant un temps d'au moins 4 à 7 semaines à compter de l'implantation.

Dans le cas où l'on utilise la membrane pour la régénération osseuse, des temps de dégradation plus longs, de l'ordre de 3 à 4 mois par exemple, sont nécessaires, ce qui sera obtenu grâce à des teneurs peu élevées en PLA de faible masse moléculaire. Il convient enfin de noter que la vitesse de dégradation des membranes n'augmente pas nécessairement lorsque l'épaisseur diminue.

Les exemples suivants illustrent l'invention.

## EXEMPLE 1 : Préparation de membrane

a) *Préparation d'un poly(acide lactique) de masse moléculaire élevée*

On effectue une polymérisation en masse de D,L-lactide en ballon scellé à 140°C, en présence de 0,5 % en poids de zinc catalyseur, en agitant, pendant 3 semaines. Après refroidissement, on dissout le produit réactionnel dans le chloroforme et on ajoute de l'éthanol pour précipiter le polymère formé, tandis que les monomères résiduels et les poly(acides lactiques) de bas poids moléculaires restent en solution.

Le polymère précipité est ensuite redissous dans l'acétone.

Il présente les caractéristiques suivantes :

$$\overline{M}_n = 160.000$$

Indice de polymolécularité : 1,7.

b) *Préparation d'un poly(acide lactique) de faible masse moléculaire*

On prépare une solution aqueuse a 85 % d'acide D,L-lactique. On distille très lentement l'eau présente dans le milieu, sous pression réduite (100 mmHg, soit 13332 Pa) puis la température est augmentée progressivement jusqu'à 120°C. On abaisse alors graduellement la pression jusqu'à 20 mmHg, soit 2666 Pa, en 6 heures à 120°C. La température est ensuite portée à 140°C et la pression maintenue à la valeur précédemment indiquée pendant une journée. Le produit obtenu au refroidissement a une consistance de pâte collante. On le solubilise dans l'acétone, à raison de 200 g par litre d'acétone. La solution obtenue est ajoutée goutte à goutte à 10 litres d'eau en agitant. On obtient un précipité de poly(acide lactique) sous la forme d'une masse visqueuse que l'on lave à l'eau plusieurs fois puis sèche sous pression réduite.

Le polymère obtenu présente les caractéristiques suivantes :

$$\overline{M}_p = 2.500$$

Indice de polymolécularité : 1,5.

c) *Préparation d'une membrane*

On a préparé des membranes de poly(acide lactique), ou PLA, contenant le polymère obtenu en a) ci-dessus ainsi que des quantités variables de polymère de faible masse moléculaire obtenu en b). Les polymères sont dissous dans l'acétone. La solution est soumise à une filtration stérilisante sur filtre Millipore 0,22 μm. Le mélange est coulé dans une boîte de Petri stérile. On évapore le solvant d'abord sous pression atmosphérique pour éviter la formation de bulles à la surface du film puis sous pression réduite (10 mmHg soit 1333 Pa) pendant 1 mois. On a ainsi obtenu des mélanges contenant 10 %, 20 % et 30 % de PLA de faibles masses moléculaires.

## EXEMPLE 2 :

Les mélanges de l'exemple 1 sont obtenus sous forme de films ayant une épaisseur de 0,3 mm.

Par calandrage à chaud, au-dessus de la température de ramollissement, on a préparé diverses membranes ayant des épaisseurs de 250 μm.

On a procédé à une étude de biocompatibilité et de résorbabilité des membranes chez le rat.

Les diverses membranes découpées à des dimensions de 4 mm x 2 mm, sont implantées soit en position sous-cutanée dans la paroi abdominale, soit en position sous-muqueuse au contact du maxillaire inférieur dans les conditions connues utilisées pour la régénération tissulaire guidée dans le traitement des maladies parodontales.

Les membranes sont maintenues en place à l'aide de points de suture en Vicryl. Un examen histologique sur coupes sériées a été pratiqué à divers intervalles de temps.

Les membranes ont été bien tolérées après 35, 50 et 60 jours d'implantation. Les seules cellules inflammatoires observées sont situées autour des points de suture en Vicryl. Pour une membrane-témoin ne contenant pas de PLA de faibles masses moléculaires (MM), on n'observe aucun signe de résorption après 60 jours d'implantation. Pour les membranes contenant 10 % de PLA de faibles MM, on note un début de résorption au bout de 60 jours. La résorption est plus avancée au bout de la même période pour les membranes contenant 20 % de PLA de faibles MM. Ce sont les membranes contenant 30 % de PLA de faibles MM qui présentent à 60 jours les signes de résorption les plus avancés : un tissu conjonctivo-vasculaire, riche en cellules, remplace progressivement l'espace occupé par les membranes à mesure de leur dégradation. Un tissu conjonctif fibreux normal, dépourvu de cellules inflammatoires, est observé par la suite.

## EXEMPLE 3 : Régénération tissulaire guidée chez le chien

Sous anesthésie générale, un lambeau gingival muco-périosté est décollé, après incision, de façon à découvrir les racines des 2e, 3e et 4e prémolaires et de la première molaire mandibulaire. Des lésions artificielles sont réalisées à l'aide d'une fraise : l'os alvéolaire vestibulaire est ainsi éliminé sur 6 mm de hauteur et 3 mm de large, de même que le cément radiculaire. En outre, une encoche est réalisée avec la fraise à la surface de la racine, au niveau le plus apical de la lésion osseuse et cémentaire. Une membrane est placée depuis une zone de la couronne voisine de la jonction émail-cément jusqu'à l'os alvéolaire résiduel. La membrane est maintenue en place par des sutures en Vicryl. Le lambeau muco-périosté est remis en place et maintenu à l'aide de points de suture. Des études histologiques ont été effectuées au bout de 42 jours, 49 jours et 90 jours. Des biopsies en bloc ont été prélevées et un examen histologique a été réalisé sur coupes sériées. Chez les animaux témoins n'ayant pas reçu d'implants, la cicatrisation a conduit à la formation d'un long épithélium, avec récidive de poches parodontales et un très léger gain de tissu osseux alvéolaire et de cément. Dans les cas où une membrane était implantée, un important gain de cément et d'os alvéolaire a été observé, avec reconstitution du ligament et du parodonte marginal.

**EXEMPLE 4** : **Régénération tissulaire guidée chez l'homme**

A la suite des essais positifs de biocompatibilité chez le rat et chez le chien beagle, des membranes contenant 30 % de PLA de faibles MM ont été implantées au niveau de lésions parodontales avancées de parodontites chroniques, avec importante destruction de l'os alvéolaire et mobilité dentaire importante, chez des patients ayant donné leur consentement éclairé. Les premiers résultats cliniques obtenus au bout de 3 mois sont très satisfaisants, avec absence de phénomènes cliniques inflammatoires.

**Revendications**

1. Utilisation, dans la préparation d'une membrane implantable biorésorbable à base de poly(acide lactique), destinée à favoriser la régénération du parodonte et/ou des maxillaires selon la technique de régénération tissulaire guidée, d'un mélange d'un premier poly(acide lactique) amorphe ayant une masse moléculaire au moins égale à 20.000 et d'un second poly(acide lactique) amorphe ayant une masse moléculaire non supérieure à 5.000, ledit mélange comportant une proportion suffisante dudit second poly(acide lactique) pour que la membrane ait le degré de souplesse désiré à une température non supérieure à 37°C.

2. Utilisation selon la revendication 1, caractérisée par le fait que lesdits premier et second poly(acides lactiques) sont constitués d'un mélange de motifs dérivés des acides D- et L-lactiques, la proportion de motifs D-lactiques étant au moins suffisante pour que lesdits poly(acides lactiques) soient amorphes.

3. Utilisation selon la revendication précédente, caractérisée par le fait que lesdits premier et second poly(acides lactiques) contiennent de 30 à 70 % de motifs D-lactiques.

4. Utilisation selon la revendication précédente, caractérisée par le fait que lesdits premier et second poly(acides lactiques) contiennent en proportions égales des motifs dérivés des acides D- et L-lactiques.

5. Utilisation selon l'une quelconque des revendications précédentes, caractérisée par le fait que ledit mélange contient de 10 à 60 % en poids dudit second poly(acide lactique).

6. Utilisation selon la revendication 5, caractérisée par le fait que l'on règle la composition dudit mélange en fonction de la vitesse de résorption souhaitée pour la membrane implantée, étant entendu que la résorption est d'autant plus rapide que la proportion du second poly(acide lactique) est plus importante et que sa masse moléculaire est plus faible.

7. Utilisation selon la revendication 5, dans la préparation d'une membrane destinée à la régénération du parodonte, caractérisée par le fait que ledit mélange contient de 20 à 60 %, et en particulier de 20 à 50 % en poids dudit second poly(acide lactique).

8. Utilisation selon la revendication précédente, caractérisée par le fait que ledit mélange contient de 25 à 50 % en poids dudit second poly(acide lactique).

9. Utilisation selon la revendication 5, dans la préparation d'une membrane destinée à la régénération des maxillaires, caractérisée par le fait que le mélange contient de 10 à 20 % en poids dudit second poly(acide lactique),.

10. Utilisation selon l'une quelconque des revendications précédentes, caractérisée par le fait que l'on prépare ladite membrane sous la forme d'un film ayant une épaisseur pouvant aller de 0,05 à 0,5 mm, et en particulier de 0,1 à 0,3 mm.

**Claims**

1. Use, in the preparation of a poly(lactic acid)-based bioresorbable implantable membrane, intended to enhance the regeneration of the periodontal tissue and/or of the maxillae according to the technique of guided tissue regeneration, of a mixture of a first amorphous poly(lactic acid) having a molecular mass of at least 20,000, and of a second amorphous poly(lactic acid) having a molecular mass not exceeding 5000, the said mixture comprising a sufficient proportion of the said second poly(lactic acid) so that the membrane has the desired degree of flexibility at a temperature not exceeding 37°C.

2. Use according to Claim 1, characterized by the fact that the said first and second poly(lactic acids) consist of a mixture of units derived from D- and L-lactic acids, the proportion of D-lactic units being at least sufficient for the said poly(lactic acids) to be amorphous.

3. Use according to the preceding claim, characterizad by the fact that the said first and second poly(lactic acids) contain 30 to 70% of D-lactic units.

4. Use according to the preceding claim, characterized by the fact that the said first and second poly(lactic acids) contain, in equal proportions, units derived from D- and L-lactic acids.

5. Use according to any one of the preceding claims, characterized by the fact that the said mixture contains 10 to 60% by weight of the said second poly(lactic acid).

6. Use according to Claim 5, characterized by the fact that the composition of the said mixture is adjusted as a function of the desired rate of resorption for the implanted membrane, it being understood that the resorption is more rapid the higher the proportion of the second poly(lactic acid) and the lower its molecular mass.

7. Use according to Claim 5, in the preparation of a membrane intended for the regeneration of the periodontal tissue, characterized by the fact that the said mixture contains from 20 to 60%, and in particular from 20 to 50% by weight of the said second poly(lactic acid).

8. Use according to the preceding claim, characterized by the fact that the said mixture contains from 25 to 50% by weight of the said second poly(lactic acid).

9. Use according to Claim 5, in the preparation of a membrane intended for the regeneration of the maxillae, characterized by the fact that the mixture contains from 10 to 20% by weight of the said second poly(lactic acid).

10. Use according to any one of the preceding claims, characterized by the fact that the said membrane is prepared in the form of a film having a thickness which may range from 0.05 to 0.5 mm, and in particular from 0.1 to 0.3 mm.

**Patentansprüche**

1. Verwendung eines Gemisches aus einer ersten amorphen Polymilchsäure mit einem Molekulargewicht von mindestens 20.000 und einer zweiten amorphen Polymilchsäure mit einem Molekulargewicht von nicht mehr als 5.000 zur Herstellung einer implantierbaren bioresorbierbaren Membran auf Basis von Polymilchsäure, die zum Fördern der Regenerierung des Zahnfleischs und/oder der Kieferknochen mit Hilfe der Technik der gesteuerten Gewebe-Regenerierung bestimmt ist, wobei das Gemisch einen solchen Anteil der zweiten Polymilchsäure enthält, der ausreicht, um der Membran den gewünschten Weichheitsgrad bei einer Temperatur von nicht mehr als 37°C zu verleihen.

2. Verwendung nach Anspruch 1, dadurch gekennzeichnet, daß die erste und die zweite Polymilchsäure aus einem Gemisch aus von D- und L-Milchsäure abgeleiteten Einheiten gebildet ist, wobei der Anteil der D-Milchsäure-Einheiten ausreichend ist, um die Polymilchsäuren amorph zu machen.

3. Verwendung nach dem vorhergehenden Anspruch, dadurch gekennzeichnet, daß die erste und die zweite Polymilchsäure 30 bis 70% an D-Milchsäure-Einheiten enthalten.

4. Verwendung nach dem vorhergehenden Anspruch, dadurch gekennzeichnet, daß die erste und die zweite Polymilchsäure gleiche Anteile an von D- und L-Milchsäure abgeleiteten Einheiten enthalten.

5. Verwendung nach einem der vorhergehenden Ansprüche, dadurch gekennzeichnet, daß das Gemisch 10 bis 60 Gew.-% der zweiten Polymilchsäure enthält.

6. Verwendung nach Anspruch 5, dadurch gekennzeichnet, daß man die Zusammensetzung des Gemisches in Abhängigkeit von der gewünschten Resorptionsgeschwindigkeit der implantierten Membran regelt, wobei berücksichtigt wird, daß die Resorption um so rascher ist, je größer der Anteil der zweiten Polymilchsäure ist und je niedriger ihr Molekulargewicht ist.

7. Verwendung nach Anspruch 7, zur Herstellung einer für die Regenerierung des Zahnfleischs bestimmten Membran, dadurch gekennzeichnet, daß das Gemisch 20 bis 60 Gew.-%, insbesondere 20 bis 50 Gew.-% der zweiten Polymilchsäure enthält.

8. Verwendung nach dem vorhergehenden Anspruch, dadurch gekennzeichnet, daß das Gemisch 25 bis 50 Gew.-% der zweiten Polymilchsäure enthält.

9. Verwendung nach Anspruch 5 zur Herstellung einer zur Regenerierung der Kieferknochen bestimmten Membran, dadurch gekennzeichnet, daß das Gemisch 10 bis 20 Gew.-% der zweiten Polymilchsäure enthält.

10. Verwendung nach einem der vorhergehenden Ansprüche, dadurch gekennzeichnet, daß die Membran in Form einer Folie einer Dicke von 0,05 bis 0,5 mm, insbesondere 0,1 bis 0,3 mm, ausgebildet wird.